# EUROPEAN PATENT SPECIFICATION

(11) **EP 2 997 360 B1**
(45) Date of publication and mention of the grant of the patent: **24.07.2024**
(21) Application number: 14729727.9
(22) Date of filing: 13.05.2014
(51) Int. Cl.: G01N 31/22, G01N 33/00

(54) **DISPOSABLE AND DISPERSIBLE EXPLOSIVE DETECTION DEVICE AND METHOD OF SIMULTANEOUS DETECTION OF EXPLOSIVES**
AUFTEILBARER WEGWERF-DETEKTOR UND VERFAHREN ZUM SIMULTANEN NACHWEIS VON SPRENGSTOFFEN
DISPOSITIF DE CAPTEUR JETABLE ET DIVISIBLE, ET PROCÉDÉ POUR LA DÉTECTION D'EXPLOSIVES.

(30) Priority: 14.05.2013 IN 1429DE2013
(43) Date of publication of application: 23.03.2016
(73) Proprietor: The Director General, Defence Research & Development Organisation (DRDO), New Delhi 110105 (IN)
(72) Inventor: NAYAK, Satish Ramdas, Pune 411021 (IN); ROY, Reny Mammen, Pune 411021 (IN); SINHA, Rabindra Kumar, Pune 411021 (IN); BHATTACHARYA, Bikash, Pune 411021 (IN); ASTHANA, Shri Nandan, Pune 411021 (IN)
(74) Representative: AWA Sweden AB
(86) International application number: PCT/IB2014/061400
(87) International publication number: WO 2014/184736

(56) References cited:
- EP-A2- 0 264 252
- EP-A2- 0 586 125
- WO-A1-2006/085955
- WO-A2-2010/086834
- CN-A- 102 507 564
- US-A- 3 552 926
- US-A1- 2005 101 027

## Description

### FIELD OF THE INVENTION

The present invention relates to a method and utility device for detecting explosives selected from inorganic nitrates like ammonium nitrate (AN), chlorates, nitramines like cyclo trimethylene trinitramine (RDX), Her Majesty's Explosive (HMX), tetryl (CE), nitrate esters like pentaerythritol tetranitrate (PETN), nitro compounds like trinitrotoluene (TNT) plastic explosives like Semtex, TATB and combinations thereof. It also relates to two variants of explosive detection kit viz. a readily portable miniaturised disposable drop type variant and a dispersible spray type variant.

### BACKGROUND OF THE INVENTION

Detection of explosives for security has been an area of global concern since decades. Concerted efforts have been focused on the detection of explosives which is a vitally challenging task, as the criminal use of explosives and improvised explosive devices (IEDs) by anti-social elements for the destruction of public life and property is increasing day by day.

The threat of IEDs has proliferated to civilian areas in addition to military installations. A combination thereof, consisting of more than one group of basic explosive chemicals, or explosives diluted with plasticizers and polymers often referred to as plastic explosives are used in IEDs known, and are designed for large scale destruction and casualties.

Contemporary methods of detection and identification are designed primarily to detect metals and thereof fail to detect explosives and IEDs not containing metallic parts. Radiographic methods fail to detect modern explosive devices. No single technique that gives a comprehensive solution to meet the operational criteria has been found till date. The compelling need of the hour demands techniques which are highly sensitive, quick, non-intrusive and mobile in nature. This necessitates a method of detecting a wide range of explosives by a single method.

Trained canine have been used since long for detection of explosives. The disadvantage of this method of known art is that dogs require an elaborate and expensive system of training repeatedly. Dogs also get distracted easily due to various factors and are not always reliable. Moreover dogs cannot identify the group of explosive chemicals or individual explosive chemical used in the manufacture of such explosive devices and/or objects. This makes it difficult to detect the explosive in suspicious objects effectively.

Another method of identification of explosives is use of explosive detectors. The disadvantage of this method is that known explosive detectors capable to detect all the classes or devices of explosives are highly expensive which limits their wide application in a country of gigantic size. Still another drawback of such detectors is that these are electronics, dependent on batteries requiring repeated charging. Moreover, these detectors have unforeseen failures in certain specific environmental conditions making them unreliable for field use.

Lloyd FJ, Forensic Science Society 7,198 (1976) describes a chemical test kit in which a suspect material is moistened with sodium hydroxide, dried and spotted with Griess' reagent, whereby a purple coloration is given by nitrate esters. This kit is not advantageous for field use as results are not obtained instantly and cannot identify/detect other classes of explosives.

Fisco BW, American Journal of Forensic Sciences 19, p 141 (1974) describes a portable explosives identification kit using many different components like miniaturized TLC plate, developing chamber, visualizer, and many complicated accessories, which makes it unsuitable for field use.

Sweeney T et al US Army Land Warfare Laboratory, Technical Report No LWL-CR-24C74, June, 1974, describes the detection of a nitrate ester by oxidizing action of nitric acid liberated by hydrolysis of the nitrate esters and colour reactions with starch-KI. The reaction is accomplished on filter paper under a heating lamp for two minutes. Therefore, the kit is not simple for field use and has limited applications.

Indian Patent No 194622 dated 08-03-1996 describes a chemical kit in which all the tests require acetone as solvent. Further, this kit has various other disadvantages like it is not disposable and cannot be carried in the pocket of the user. Dispersible variant is not available. Hence reagents cannot be sprayed directly on suspect material. Concentrated sulphuric acid is used in the kit which is a very hazardous chemical that causes instant burning in case of skin contact. Ceramic test plates in the kit are heavy and inconvenient for user.

Sulphuric acid is capable of causing very severe burns, especially when it is at a high concentration. In common with other corrosive strong acids and strong alkalis, it results in chemical burns upon contact as it readily decomposes proteins and lipids in living tissues through amide hydrolysis and ester hydrolysis. Besides this, it also exhibits a strong dehydrating property which dehydrates carbohydrates, liberating extra heat and leading to additional secondary thermal burns. Its strong oxidizing property may extend its corrosiveness as well. Because of such reasons, damage caused by sulphuric acid is potentially more serious than that caused by many other comparable strong acids, such as hydrochloric acid and nitric acid. Consequently, it rapidly attacks cornea if splashed onto eyes which can induce permanent blindness and it causes irreversible destruction to internal organs or may even be fatal if swallowed.

There is a clear need to develop a kit which is safe, disposable, cost effective, portable, light weight, simple to operate, fast, is always in a ready to use condition and that can be used readily in field conditions for immediate detection and identification of a wide range of explosives. The present inventors have surprisingly developed a method and kit that ameliorates the shortcomings of the prior art.

### OBJECT OF THE INVENTION

It is an object of the present invention to provide a method for detecting explosives selected from inorganic nitrates like ammonium nitrate (AN), chlorates, nitramines like cyclo trimethylene trinitramine (RDX), cyclo tetramethylene tetranitramine (HMX), tetryl (CE), nitrate esters like pentaerythritol tetranitrate (PETN), nitro compounds like trinitrotoluene (TNT) plastic explosives like Semtex, TATB and combinations thereof. The method of the present invention is defined in claim 2.

It is yet another object of the present invention to provide a readily portable miniaturised explosive detection utility device that is disposable drop type, to react the suspected sample on sample test plate. The utility device of the present invention is defined in claim 1.

It is yet another object of the present invention to provide an explosive detection utility device that is dispersible spray type to provide a stand off in case of suspected sample.

Typically an explosive detection kit is provided that is disposable, cost effective, portable, safe for transport and use, light weight, simple to operate, fast, is always in a ready to use condition, can be used readily in field conditions and identifies not only one group of the explosive chemicals at one time but also individual explosive chemicals of different classes by formation of their particular specific colour derivatives.

The chemical reaction for explosive detection and identification can be carried out on a high density polyethylene (HDPE) test plate or directly on suspected object contaminated with explosive chemicals in both lab and field conditions in the shortest sequence of chemical spot reactions under preferably all environmental conditions.

### SUMMARY OF THE INVENTION

According to an aspect of the present invention there is provided a portable, disposable explosive detection utility device comprising:
(a) Multiple compartments for housing reagent tubes for detecting explosives;
(b) a compartment for housing a pin;
(c) multiple test beds for detection of explosives; and
(d) a colour chart to identify the explosives.

According to another aspect of the present invention there is provided a dispersible explosive detection utility device comprising:
(a) Multiple high density polyethylene spray bottles for detecting explosives;
(b) a test plate for individual or simultaneous detection of the explosives; and
(c) a colour chart to identify the explosives.

### BRIEF DESCRIPTION OF DRAWINGS

Figure 1: Top view of the Disposable Drop type Explosive Detection Kit
Figure 2a: Outer view of the High Density Polyethylene Spray Bottle (Filled)
Figure 2b: Internal view of the High Density Polyethylene Spray Bottle (Filled)

### DESCRIPTION OF THE INVENTION

The present invention provides the method and a utility device for the detection of explosives such as inorganic nitrates like ammonium nitrate (AN), chlorates, nitramines like cyclo trimethylene trinitramine (RDX), Her Majesty's Explosive (HMX), tetryl (CE), nitrate esters like pentaerythritol tetranitrate (PETN), nitro compounds like trinitrotoluene (TNT) and plastic explosives like Semtex, TATB and combinations thereof.

The explosive detection kit (EDK) comprises multiple reagents, reacted individually with the sample of explosive chemicals drawn from exploded and/or unexploded site, and resulting in formation of specific colour derivative of the suspected explosive chemical, based on the specific chemical reaction between the individual specific reagent of the kit and the sample from the suspected site.

In the utility device of the present invention, an explosive detection kit with multiple reagents is provided wherein:
1. The first reagent for detection of nitramine (RDX) /nitramine based plastic explosive comprises Tetra butyl ammonium hydroxide approximately 4 - 6ml, 25 - 35ml of dimethyl sulphoxide, 6 - 10ml methanol, 0.1 - 0.22g N-(1-naphthyl) ethylenediamine dihydrochloride and 2.5 - 3.2g sulphanilamide in 5 - 10% aqueous phosphoric acid. This reagent on reacting with nitramine (RDX) /nitramine based plastic explosive gives magenta colour instantly.
2. The second reagent for detection of nitrates comprises 1- 2.5g sulfanilic acid with 50 - 70m1 glacial acetic acid and 0.1 - 0.35g N-(1-naphthyl) ethylenediamine dihydrochloride and 0.5 - 2.5g zinc dust This reagent on reaction with nitrates gives dark pink colour.
3. The third reagent for detection of chlorates comprises 4 - 6g sulfanilic acid, 9 - 15m1 DMSO, 10 - 25ml methanol, 50 - 70ml distilled water and 2.3 - 4.7g aniline sulphate This reagent on reaction with chlorates gives blue colour.
4. The fourth reagent for detection of TNT comprises tetra butyl ammonium hydroxide approximately 4 - 7ml, with 25 - 50ml of dimethyl sulphoxide and 6 - 15m1 methanol and 0.1 - 0.35g N-(1-naphthyl) ethylenediamine dihydrochloride. This reagent on reaction with TNT gives maroon colour.
5. The fifth reagent for detection of CE comprises 1 - 3.5g sulfanilic acid with 50 - 70ml glacial acetic acid and 1 - 3.5g N-(1-naphthyl) ethylenediamine dihydrochloride and 0.5-1.5g zinc dust. This reagent on reaction with CE gives tomato red colour.
6. The sixth reagent for detection of TATB comprises 2.5 - 5.5g Potassium hydroxide, 5 - 20ml distilled water and 45 - 75ml DMSO. This reagent on reaction with TATB gives yellowish orange colour.
7. The seventh reagent for detection of PETN comprises 3 - 4.5g sulfanilic acid with 50 - 70ml glacial acetic acid and 0.3 - 4.2g N-(1-naphthyl) ethylenediamine dihydrochloride and 0.5 - 1.5g zinc dust. This reagent on reaction with PETN gives purple pink colour

According to preferred methodology of the present invention the starting reactant in pure/contaminated form is divided into batches, preferably each of 2 mg to 10 mg and allowed to react individually with each of the reagent of the kit on any surface, particularly on high density polyethylene (HDPE) test plate provided in the kit. Each batch is allowed to react individually and simultaneously with not more than one reagent of the proposed kit, for preferably two minutes, to yield the specific colour derivative of the suspected explosive chemical that is used to identify the suspected explosive chemical.

The advantages of the explosive utility device of the present invention and the method of reaction of the said reactants of the said kit are that the whole chemical process for explosive detection and identification completes simultaneously on all the batches of the starting reactant within few minutes preferably upto maximum of two minutes under all environmental conditions, involving chemical reaction in each batch of suspected reactant and the reagent of the proposed kit, hence the process being highly time saving, convenient and reliable. The further objects, embodiments and advantages of the present invention will be more apparent from the following working examples of the present invention when read in conjunction with the foregoing description.

An explosive detection kit (EDK) is provided that is disposable drop type wherein the disposable EDK(DEDK) comprises seven reagents, which are allowed to react individually, separately and simultaneously with the sample of explosive chemicals in separate batches drawn from exploded and/or unexploded site, and resulting in formation of specific colour derivative of the suspected explosive chemical, based on the specific chemical reaction between the individual specific reagent of the kit and the sample from the suspected site under all environmental conditions. Disposable Explosive Detection Kit (DEDK) uses reagents which are suitable for transport by all modes and can be commercially marketed.

DEDK is light weight, portable, safe for transport, ready to use, simple to operate, gives immediate results for pure explosives as well as in contaminated conditions. DEDK is user friendly and can be operated by unskilled personnel also, as operational instructions are provided in the DEDK giving accurate results. Test beds have been designed on the lid of the DEDK made of HDPE material. All tests can be performed and colours developed are compared with colour chart provided in the DEDK.

According to another preferred embodiment of the present invention an explosive detection utility device is provided that is dispersible spray type and provides a stand off in case of suspected sample. The advantage of the aerosol based explosive detection kit is reagents can be sprayed on suspect material without retrieving on test plate and is also suitable for bulk detection.

### EXAMPLES

By the following examples, the present invention would be exemplified and explained concretely. Needless to say, the same may not be construed as delimitation of the scope of the invention thereto.

### Example 1

### Reagent for detection of nitramine (RDX) /nitramine based plastic explosive

The first reagent for detection of nitramine (RDX) /nitramine based plastic explosive comprises Tetra butyl ammonium hydroxide approximately 4 - 6ml, 25 - 35ml of dimethyl sulphoxide, 6 - 10ml methanol, 0.1 - 0.22g N-(1-naphthyl) ethylenediamine dihydrochloride and 2.5 - 3.2g sulphanilamide in 5 - 10% aqueous phosphoric acid. This reagent on reacting with nitramine (RDX) /nitramine based plastic explosive gives magenta colour instantly.

### Example 2

### Reagent for detection of nitrates

The second reagent for detection of nitrates comprises 1- 2.5g sulfanilic acid with 50 - 70ml glacial acetic acid and 0.1 - 0.35g N-(1-naphthyl) ethylenediamine dihydrochloride and 0.5 - 2.5g zinc dust This reagent on reaction with nitrates gives dark pink colour.

### Example 3

### Reagent for detection of chlorates

The third reagent for detection of chlorates comprises 4 - 6g sulfanilic acid, 9 - 15ml DMSO, 10 - 25ml methanol, 50 - 70ml distilled water and 2.3 - 4.7g aniline sulphate .This reagent on reaction with chlorates gives blue colour.

### Example 4

### Reagent for detection of TNT

The fourth reagent for detection of TNT comprises tetra butyl ammonium hydroxide approximately 4 - 7ml, with 25 - 50ml of dimethyl sulphoxide and 6 - 15ml methanol and 0.1 - 0.35g N-(1-naphthyl) ethylenediamine dihydrochloride. This reagent on reaction with TNT gives maroon colour.

### Example 5

### Reagent for detection of CE

The fifth reagent for detection of CE comprises 1 - 3.5g sulfanilic acid with 50 - 70ml glacial acetic acid and 1 - 3.5g N-(1-naphthyl) ethylenediamine dihydrochloride and 0.5-1.5g zinc dust. This reagent on reaction with CE gives tomato red colour.

### Example 6

### Reagent for detection of TATB

The sixth reagent for detection of TATB comprises 2.5 - 5.5g Potassium hydroxide, 5 - 20ml distilled water and 45 - 75ml DMSO. This reagent on reaction with TATB gives yellowish orange colour.

### Example 7

### Reagent for detection of PETN

The seventh reagent for detection of PETN comprises 3 - 4.5g sulfanilic acid with 50 - 70ml glacial acetic acid and 0.3 - 4.2g N-(1-naphthyl) ethylenediamine dihydrochloride and 0.5 - 1.5g zinc dust. This reagent on reaction with PETN gives purple pink colour

### Example 8

### Disposable Explosive Detection Kit (DEDK)

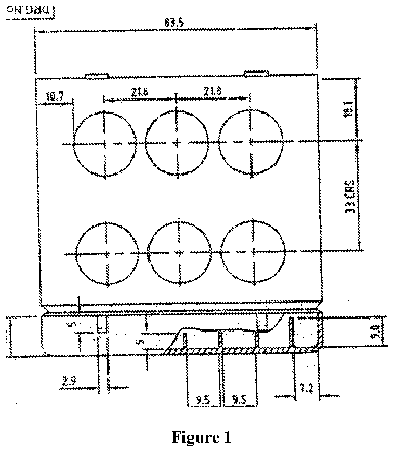

A particular embodiment of the disposable kit in accordance with the present invention as illustrated in Figure 1 is a miniaturised disposable explosive detection kit (DEDK) made of high density polyethylene (HDPE). The DEDK box consists of eight compartments wherein seven compartments are for housing reagent tubes and one compartment for housing a SS pin (for opening the sealed tube). 2ml of reagent is provided in each reagent tube. Each tube and its cap are made of HDPE. The lid of the box is made of six test beds which are also made of HDPE.

The following test procedure is carried out: The suspected exploded/unexploded sample comprising of explosive prepared according to the known procedure is divided into 6 batches of 2-10 mg each taken in inner lid of disposable kit which houses the test beds and reagents are allowed to react with the samples individually.

### Example 9

### Dispersible Explosive Detection Kit

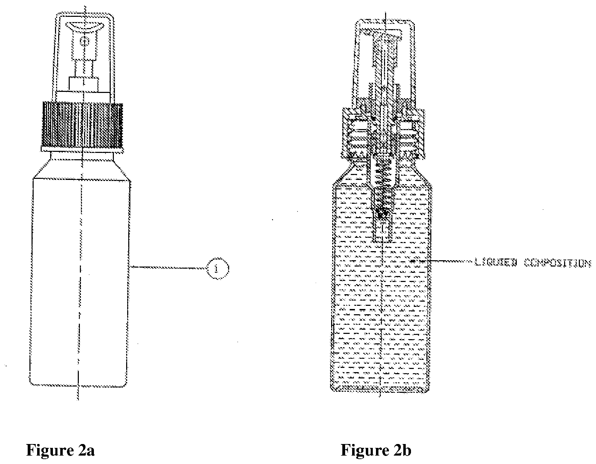

A particular embodiment of the dispersible kit in accordance with the present invention is an aerosol based detection kit that consists of aerosol spray pump, aerosol spray sleeve, spray tube, pressing cap, comprising of spring, ball, grip washer, 'O' ring, bush, top cover and holder cap which are made of HDPE. Figure 2a and figure 2b illustrate the high density polyethylene spray bottle (filled) with the reagents. Seven spray bottles are kept vertically in the specially designed box to hold the bottles upright (vertically).The volume of reagent in each bottle is 50 ml. The said kit also comprises colour chart, operational manual, and test plates.

The following test procedure is carried out: The suspected exploded/unexploded sample comprising of explosive prepared according to the known procedure is divided into seven batches of 2-10 mg each, taken on the HDPE test plate and reagents present in the spray bottle are sprayed to react with the samples individually.

### Example 10

### Working Example

3 to 4 drops of reagent 1, 2, 3, 4, 5, 6, 7 are allowed to react individually with different samples taken in the test plate. The colour visible on reaction is compared with the colour chart as provided in Table 1 with respect to each explosive for its identification in the suspected sample. The yield of magenta derivative on reaction of Reagent 1 solution with suspected sample indicates presence of RDX/ RDX based plastic explosive. The yield of dark pink derivative on reaction of Reagent 2 with suspected sample, confirms presence of ammonium nitrate/inorganic nitrates. The yield of blue derivative on reaction of Reagent 3 with suspected sample indicates presence of chlorates. The yield of maroon derivative on reaction of Reagent 4 with suspected sample indicates presence of TNT. The yield of tomato red derivative on reaction of Reagent 5 with suspected sample, confirms presence of tetryl (CE). The yield of yellowish orange derivative on reaction of Reagent 6 with suspected sample, confirms presence of TATB. The yield of purple pink derivate on reaction of Reagent 7 indicates presence of PETN. It is clear from the foregoing examples that the suspected explosives based on, RDX/ RDX based plastic explosives, inorganic nitrates, chlorates, TNT, CE, TATB, and PETN explosive could be completely detected by DEDK in not more than two minutes.

**Table 1: Explosive Detection Colour Chart**

| **Explosive name** | **Colour indicator** |
|---|---|
| Nitramine(RDX) / nitramine based plastic explosive | Magenta |
| Nitrates | Dark pink |
| Chlorates | Blue |
| TNT | Maroon |
| CE | Tomato red colour |
| TATB | Yellowish orange |
| PETN | Purple pink |

## Claims

1. A portable, disposable explosive detection utility device comprising:
- 7 sealed reagent tubes each comprising a reagent for detecting an explosive;
- a pin for opening said sealed reagent tubes;
- multiple test beds for detection of explosives; and
- a colour chart to identify the explosives,
wherein said reagents are selected from the group:
a. Reagent 1 comprising 4 - 6ml of Tetra butyl ammonium hydroxide, 25 - 35ml of dimethyl sulphoxide, 6 - 10ml methanol, 0.1 - 0.22g N-(1-naphthyl) ethylenediamine dihydrochloride and 2.5 - 3.2g sulphanilamide in 5 - 10% aqueous phosphoric acid,
b. Reagent 2 comprising 1- 2.5g sulfanilic acid with 50 - 70ml glacial acetic acid and 0.1 - 0.35g N-(1-naphthyl) ethylenediamine dihydrochloride and 0.5 - 2.5g zinc dust,
c. Reagent 3 comprising 4 - 6g sulfanilic acid, 9 - 15ml DMSO, 10 - 25ml methanol, 50 - 70ml distilled water and 2.3 - 4.7g aniline sulphate,
d. Reagent 4 comprising tetra butyl ammonium hydroxide approximately 4 - 7ml, with 25 - 50ml of dimethyl sulphoxide and 6 - 15ml methanol and 0.1 - 0.35g N-(1-naphthyl) ethylenediamine dihydrochloride,
e. Reagent 5 comprising 1 - 3.5g sulfanilic acid with 50 - 70ml glacial acetic acid and 1 - 3.5g N-(1-naphthyl) ethylenediamine dihydrochloride and 0.5-1.5g zinc dust,
f. Reagent 6 comprising 2.5 - 5.5g potassium hydroxide, 5 - 20ml distilled water and 45 - 75ml DMSO, and
g. Reagent 7 comprising 3 - 4.5g sulfanilic acid with 50 - 70ml glacial acetic acid and 0.3 - 4.2g N-(1-naphthyl) ethylenediamine dihydrochloride and 0.5 - 1.5g zinc dust;
wherein the explosives are selected from nitramine (RDX)/nitramine based plastic explosive, nitrates, chlorates, trinitrotoluene (TNT), tetryl (CE), triaminotrinitrobenzene (TATB), pentaerythritol tetranitrate (PETN) and combinations thereof.

2. A process for individual or simultaneous detection of explosives comprising the steps of:
a. Providing a disposable explosive detection utility device as defined in claim 1;
b. Dividing the suspected explosive sample into multiple batches wherein each batch comprises of 2 mg to 10 mg sample;
c. Reacting the said sample with each reagent tube by dropwise addition;
d. Allowing the reagent to react with the sample for at least two minutes;
e. Observing the change in the colour of the sample;
f. Comparing the colour of the reacted sample with the colour chart provided in the device as defined in claim 1 to identify the explosive.

3. A dispersible explosive detection utility device comprising:
a. Multiple high density polyethylene spray bottles comprising explosive detective reagents as claimed in claim 1;
b. A test plate for individual or simultaneous detection of the explosives; and
c. A colour chart to identify the explosives.

## Patentansprüche

1. Tragbares Einweg-Sprengstoffdetektionsgerät, bestehend aus:
- 7 versiegelten Reagenzröhrchen, die jeweils ein Reagenz zum Erkennen eines Sprengstoffs umfassen;
- einer Nadel zum Öffnen der versiegelten Reagenzröhrchen,
- mehreren Testbetten zur Erkennung von Sprengstoffen und
- einer Farbkarte zur Identifizierung der Sprengstoffe,
wobei die Reagenzien aus der folgenden Gruppe ausgewählt sind:
a. Reagenz 1, umfassend 4 - 6 ml Tetrabutylammoniumhydroxid, 25 - 35 ml Dimethylsulfoxid, 6 - 10 ml Methanol, 0,1 - 0,22 g N-(1-Naphthyl)ethylendiamin-dihydrochlorid sowie 2,5 - 3,2 g Sulfanilamid in 5 - 10 %iger wässriger Phosphorsäure,
b. Reagenz 2, umfassend 1 - 2,5 g Sulfanilsäure mit 50 - 70 ml Eisessig und 0,1 - 0,35 g N-(1-Naphthyl)ethylendiamindihydrochlorid sowie 0,5 - 2,5 g Zinkstaub,
c. Reagenz 3, umfassend 4 - 6 g Sulfanilsäure, 9 - 15 ml DMSO, 10 - 25 ml Methanol, 50 - 70 ml destilliertes Wasser sowie 2,3 - 4,7 g Anilinsulfat,
d. Reagenz 4, umfassend Tetrabutylammoniumhydroxid ca. 4 - 7 ml, mit 25 - 50 ml Dimethylsulfoxid und 6 - 15 ml Methanol sowie 0,1 - 0,35 g N-(1-Naphthyl)ethylendiamindihydrochlorid,
e. Reagenz 5, umfassend 1 - 3,5 g Sulfanilsäure mit 50 - 70 ml Eisessig und 1 - 3,5 g N-(1-Naphthyl)-ethylendiamindihydrochlorid sowie 0,5 - 1,5 g Zinkstaub,
f. Reagenz 6, umfassend 2,5 - 5,5 g Kaliumhydroxid, 5 - 20 ml destilliertes Wasser sowie 45 - 75 ml DMSO, und
g. Reagenz 7, umfassend 3 - 4,5 g Sulfanilsäure mit 50 - 70 ml Eisessig und 0,3 - 4,2 g N-(1-Naphthyl)ethylendiamindihydrochlorid sowie 0,5 - 1,5 g Zinkstaub;
wobei die Sprengstoffe ausgewählt sind aus Nitramin (RDX)/Nitraminbasiertem Plastiksprengstoff, Nitraten, Chloraten, Trinitrotoluol (TNT), Tetryl (CE), Triaminotrinitrobenzol (TATB), Pentaerythrittetranitrat (PETN) und Kombinationen davon.

2. Verfahren zur individuellen oder gleichzeitigen Erkennung von Explosivstoffen, das die folgenden Schritte umfasst:
a. Bereitstellen eines Einweg-Sprengstoffdetektionsgeräts wie in Anspruch 1 definiert;
b. Aufteilen der mutmaßlichen Sprengstoffprobe in mehrere Chargen, wobei jede Charge eine Probe von 2 mg bis 10 mg umfasst;
c. Umsetzen der besagten Probe mit jedem Reagenzröhrchen durch tropfenweise Zugabe;
d. Umsetzen lassen des Reagenz mit der Probe für mindestens zwei Minuten;
e. Beobachten der Farbänderung der Probe;
f. Vergleichen der Farbe der umgesetzten Probe mit der Farbkarte, die in der Vorrichtung wie in Anspruch 1 definiert bereitgestellt wird, um den Sprengstoff zu identifizieren.

3. Einweg-Sprengstoffdetektionsgerät, umfassend:
a. mehrere Sprühflaschen aus Polyethylen hoher Dichte, die Sprengstoffdetektionsreagenzien nach Anspruch 1 umfassen;
b. eine Testplatte zur individuellen oder gleichzeitigen Erkennung der Sprengstoffe; und
c. eine Farbkarte zur Identifizierung der Sprengstoffe.

## Revendications

1. Dispositif utilitaire de détection d'explosifs portable et jetable comprenant :
- 7 tubes de réactif scellés comprenant chacun un réactif pour détecter un explosif ;
- une goupille pour ouvrir lesdits tubes de réactif scellés ;
- de multiples bancs de test pour la détection des explosifs ; et
- un nuancier pour identifier les explosifs,
dans lequel lesdits réactifs sont choisis dans le groupe :
a. Réactif 1 comprenant 4 à 6 ml d'hydroxyde de tétrabutylammonium, de 25 à 35 ml de diméthylsulfoxyde, de 6 à 10 ml de méthanol, de 0,1 à 0,22 g de dichlorhydrate de N-(1-naphtyl)éthylènediamine et de 2,5 à 3,2 g de sulfanilamide dans 5 à 10 % d'acide phosphorique aqueux,
b. Réactif 2 comprenant de 1 à 2,5 g d'acide sulfanilique avec de 50 à 70 ml d'acide acétique glacial et de 0,1 à 0,35 g de dichlorhydrate de N-(1-naphtyl)éthylènediamine et de 0,5 à 2,5 g de poussière de zinc,
c. Réactif 3 comprenant de 4 à 6 g d'acide sulfanilique, de 9 à 15 ml de DMSO, de 10 à 25 ml de méthanol, de 50 à 70 ml d'eau distillée et de 2,3 à 4,7 g de sulfate d'aniline,
d. Réactif 4 comprenant environ de 4 à 7 ml d'hydroxyde de tétrabutylammonium, avec de 25 à 50 ml de diméthylsulfoxyde et de 6 à 15 ml de méthanol et de 0,1 à 0,35 g de dichlorhydrate de N-(1-naphtyl)éthylènediamine,
e. Réactif 5 comprenant de 1 à 3,5 g d'acide sulfanilique avec de 50 à 70 ml d'acide acétique glacial et de 1 à 3,5 g de dichlorhydrate de N-(1-naphtyl)éthylènediamine et de 0,5 à 1,5 g de poussière de zinc,
f. Réactif 6 comprenant de 2,5 à 5,5 g d'hydroxyde de potassium, de 5 à 20 ml d'eau distillée et de 45 à 75 ml de DMSO, et
g. Réactif 7 comprenant de 3 à 4,5 g d'acide sulfanilique avec de 50 à 70 ml d'acide acétique glacial et de 0,3 à 4,2 g de dichlorhydrate de N-(1-naphtyl)éthylènediamine et de 0,5 à 1,5 g de poussière de zinc ;
dans lequel les explosifs sont choisis parmi les explosifs plastiques à base de nitramine (RDX)/nitramine, les nitrates, les chlorates, le trinitrotoluène (TNT), le tétryle (CE), le triaminotrinitrobenzène (TATB), le tétranitrate de pentaérythritol (PETN) et des combinaisons de ceux-ci.

2. Procédé pour la détection individuelle ou simultanée d'explosifs comprenant les étapes consistant à :
a. fournir un dispositif utilitaire de détection d'explosifs portable tel que défini dans la revendication 1 ;
b. diviser l'échantillon d'explosif suspecté en plusieurs lots, chaque lot comprenant de 2 mg à 10 mg d'échantillon ;
c. faire réagir ledit échantillon avec chaque tube de réactif par ajout goutte à goutte ;
d. laisser le réactif réagir avec l'échantillon pendant au moins deux minutes ;
e. observer le changement de couleur de l'échantillon ;
f. comparer la couleur de l'échantillon ayant réagi avec le nuancier fourni dans le dispositif tel que défini dans la revendication 1 pour identifier l'explosif.

3. Dispositif utilitaire de détection d'explosifs dispersable comprenant :
a. de multiples flacons pulvérisateurs en polyéthylène haute densité comprenant les réactifs de détection d'explosifs selon la revendication 1 ;
b. une plaque de test pour la détection individuelle ou simultanée des explosifs ; et
c. un nuancier pour identifier les explosifs.
